# EUROPEAN PATENT SPECIFICATION

(11) **EP 1 447 072 B1**
(45) Date of publication and mention of the grant of the patent: **27.07.2011**
(21) Application number: 04075468.1
(22) Date of filing: 13.02.2004
(51) Int. Cl.: A61J 1/00

(54) **Method and device for transferring fluids between sampling tubes**
Verfahren und Vorrichtung zur Flüssigkeitsübertragung zwischen Probeentnahmeröhrchen
Méthode et dispositif de transfert de fluides entre tubes de prélèvement

(30) Priority: 13.02.2003 US 365939
(43) Date of publication of application: 18.08.2004
(73) Proprietor: Becton Dickinson and Company, Franklin Lakes, New Jersey 07417 (US)
(72) Inventor: Walenciak, Matthew, Westfield, NJ 07090 (US); Augello, Frank, Cedar Knolls, NJ 07929 (US); Swenson, Kirk, North Caldwell, NJ 07006 (US)
(74) Representative: 't Jong, Bastiaan Jacob

(56) References cited:
- WO-A-86/05683
- FR-A3- 2 487 680
- US-A- 3 872 867
- US-A- 4 317 456
- US-A1- 2002 087 141

## Description

### BACKGROUND OF THE INVENTION

### 1. Field of the Invention

The present invention relates to a fluid transfer assembly. More particularly, the present invention is directed to a medical fluid transfer device that links two blood collection tube holders.

### 2. Description of Related Art

Disposable medical devices having medical needles are used for withdrawing fluid from the body of a patient. When necessary, additional medical devices having medical needles are used to transfer all or part of the withdrawn fluid from one collection container to another container. Such disposable medical devices typically include blood-collecting needles, fluid handling needles and assemblies thereof. Current medical practice requires that fluid containers and needle assemblies used in such devices be inexpensive and readily disposable. Consequently, existing blood collection and transfer devices typically employ some form of durable, reusable holder on which detachable and disposable medical needles and fluid collection tubes may be mounted. A blood collection or transfer device of this nature may be assembled prior to use and then disassembled after use. Thus, these blood collection and transfer devices allow repeated use of a relatively expensive holder upon replacement of relatively inexpensive medical needles and/or fluid collection tubes. In addition to reducing the cost of collecting and transferring blood specimens, these blood collection and transfer devices help minimize the production of hazardous waste material.

One particular example of a system for the transfer and collection of body fluids, i.e., blood, is described in U.S. Patent No. 5,360,423. This patent describes a needle assembly attached to a port guide. The needle assembly includes a needle surrounded by a piercable sheath. The needle assembly may include a female or a male connector for interconnection with a separate member, for example, a conventional syringe male fitting.

This state of the art does not provide for a convenient device that allows for the direct transfer of a fluid, i.e., blood, from one sampling container to another. Current devices and assemblies require intermediate steps to move the fluid from the first container to the second.

Accordingly, a need exists for a fluid transfer device in which fluid from one blood collection tube, is directly transferred to a second second blood collection tube.

Document WO-A-86/05683 discloses a device for transferring a substance between two vessels. The vessels are connectable to each other via a connecting member which is provided with a through cannula. The cannula is provided with a point in both ends, which points extend into a holder each. Protective sheaths surrounds the free end portions of the cannula. The connecting member has in both end portions coupling means for disconnectable coupling to the holders.

Document FR-A-2 487 680 discloses a system for preparing therapeutic solutions for infusion by piping solvent from one flask to second flask comprising a therapeutic compound. The two flasks are connected by a length of flexible tube which has a hollow needle at each end. One inserted through each of the plugs in the necks of each flask.

### SUMMARY OF THE INVENTION

The invention is defined in the independent claims 1, 11 and 13. Preferred embodiments are defined in the dependent claims.

The present invention is directed to a medical fluid transfer device as defined in claim 1 which involves the linking of two conventional blood collection sampling tube holders, or needle holders, which are typically used for blood collection and sampling procedures. The holders are arranged in opposing relation, such as with the threaded ends opposing each other, and are coupled together through a coupling unit or coupler. The coupler includes pointed needle cannulae extending from both ends of the coupler and desirably includes a piercable sleeve encompassing each of the needle cannulae. The coupler may also include structure for mating with both holders, such as external threads on both ends for threaded engagement with corresponding threads on the two holders. The coupler thereby links two holders together, with one needle cannula extending within each of the holders. The coupler according to the invention is a three-piece hub in which two hubs including separate needle cannulae are interconnected through a flexible tube-like structure.

The present invention is also directed to a method of transferring body fluids between a first sampling tube and a second sampling tube, as defined in claim 11.

In a further embodiment, the present invention is directed to a method of assembling a fluid transfer device as defined in claim 13.

### BRIEF DESCRIPTION OF THE DRAWINGS

FIG. 1 is perspective view of a fluid transfer device representing background art;

FIG. 2 is an exploded perspective view of the fluid transfer device of FIG. 1;

FIG. 3 is a side cross-sectional view of the fluid transfer device of FIG. 1;

FIG. 4 is perspective view of a fluid transfer device in accordance with an alternate embodiment of the background art;

FIG. 5 is an exploded perspective view of the fluid transfer device of FIG. 4;

FIG. 6 is a side cross-sectional view of the fluid transfer device of FIG. 4;

FIG. 7 is perspective view of a fluid transfer device in accordance with a further embodiment of the background art;

FIG. 8 is an exploded perspective view of the fluid transfer device of FIG. 7;

FIG. 9 is a side cross-sectional view of the fluid transfer device of FIG. 7;

FIG. 10 is perspective view of a fluid transfer device in accordance with a further embodiment of the background art;

FIG. 11 is an exploded perspective view of the fluid transfer device of FIG. 10;

FIG. 12 is a side cross-sectional view of a fluid transfer device of FIG. 10;

FIG. 13 is perspective view of a fluid transfer device in accordance with an embodiment of the present invention;

FIG. 14 is a side cross-sectional view of the fluid transfer device of FIG. 13; and

FIG. 15 is a side cross-sectional view of a fluid transfer device of FIG. 1 shown in use during a transfer procedure.

### DESCRIPTION OF THE PREFERRED EMBODIMENT

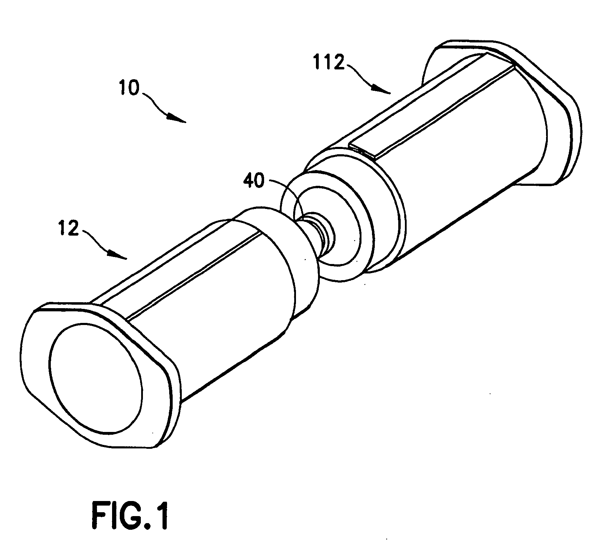

Referring to the drawings in which like reference characters refer to like parts throughout the several views thereof, FIG. 1 illustrates a fluid transfer device including a first and second sampling tube holder and a coupler that configuration does not fall within the scope of the claims. It is a blood transfer device including a coupler for linking a first sampling tube holder with a second sampling tube holder and is directed to such an assembly, as well as to the coupler itself and subassemblies incorporating the coupler, wherein the coupler comprises a flexible tube-like structure.

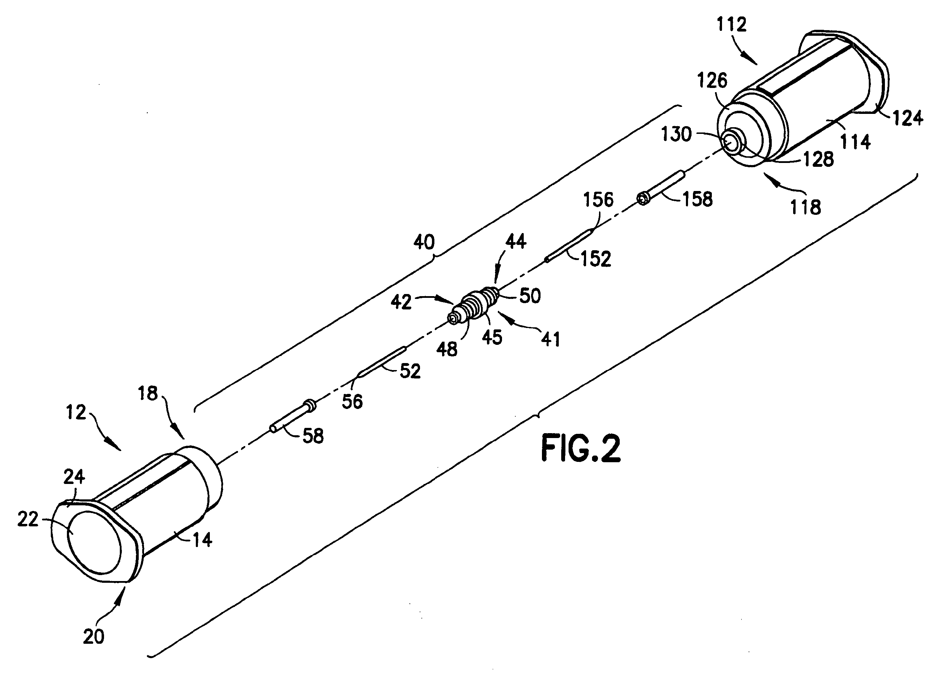

FIGS. 1-3 illustrate a fluid transfer device **10** as representation of the art, having a first sampling tube holding region such as a first sampling tube holder **12,** and a second sampling tube holder region such as a second sampling tube holder **112,** which are linked through a coupler **40.** Desirably, the first sampling tube holder region and the second sampling tube holder region are provided through a pair of connected conventional holders which are known for holding a sampling needle and a sampling tube during a phlebotomy procedure.

Each of first sampling tube holder **12** and second sampling tube holder **112** includes a generally tubular or cylindrical housing **14, 114** defined by a tubular wall **16, 116** extending between a forward end **18, 118** and a rearward end **20, 120** with an internal opening **22, 122** extending therethrough. Rearward end **20, 120** of housing **14, 114** is open-ended into internal opening **22, 122** and may include a flange **24, 124** extending perimetrically outward around the open end at rearward end **20, 120.** Forward end **18, 118** of housing **14, 114** includes a forward wall **26, 126** which extends to a shoulder to form a cylindrical neck **28, 128.** Neck **28, 128** includes an aperture, such as an opening **30, 130,** therethrough which extends into internal opening **22, 122** of housing **14, 114.** Structure or means for engagement with coupler **40, 140** to maintain such a fluid transfer device during a blood transfer procedure is further provided at forward end **18, 118.** For example, internal threads **32, 132** may be provided within the opening **30, 130** of neck **28, 128,** which are provided for threaded engagement with corresponding threads on coupler **40, 140.** Alternatively, any structure or means for engagement which is capable of connecting coupler **40, 140** to first sampling tube holder **12** and second sampling tube holder **112** may be provided, such as a snap-fit engagement, releasable engagement, and other connections.

First sampling tube holder **12** and second sampling tube holder **112** are particularly sized so as to accommodate respective first and second sampling tubes having a maximum diameter of about 16 millimeters, desirably between about 13 millimeters and 16 millimeters. First sampling tube holder **12** and second sampling tube holder **112** may be constructed of any material known in the art, desirably a polymeric material. Desirably, first sampling tube holder **12** and second sampling tube holder **112** are constructed of polypropylene. One particular example of a sampling tube holder which is particularly useful is the VACUTAINER^{®} brand holder available from Becton, Dickinson and Company of Franklin Lakes, New Jersey.

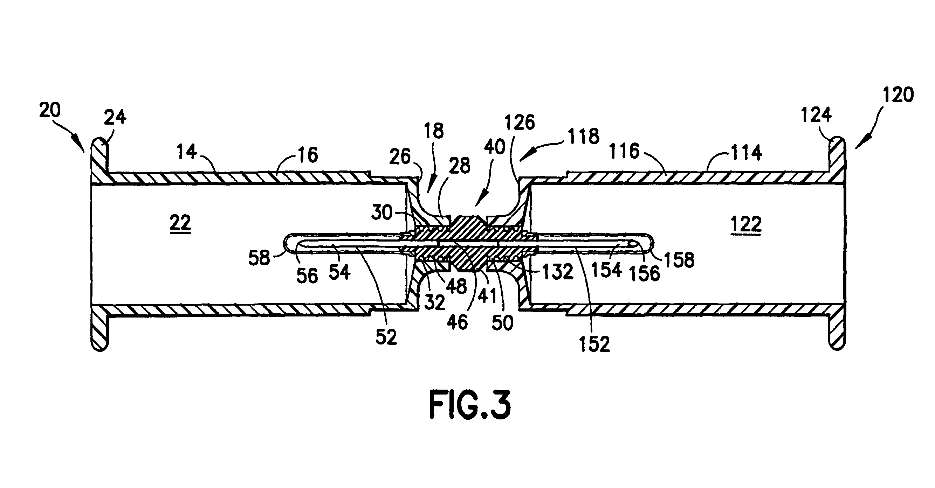

First and second sampling tube holders **12** and **112** are joined together in an end-to-end fashion through coupler **40,** as shown in FIGS. 1 and 3. Coupler **40** is defined by housing **41,** and includes a first end **42** and a second end **44** which are separated by a flange **45** therebetween, and with a passageway **46** extending therethrough establishing a path for fluid flow. Each of first end **42** and second end **44** include structure or means for engagement with first sampling tube holder **12** and second sampling tube holder **112,** respectively. For example, external threads **48, 50** may be provided at first end **42** and second end **44,** respectively, for threaded engagement with corresponding internal threads **32** of first sampling tube holder **12** and internal threads **132** of second sampling tube holder **112.** Alternatively, any structure or means for engagement which is capable of connecting coupler **40** to first sampling tube holder **12** and second sampling tube holder **112** may be provided, such as a corresponding surface for snap-fit engagement, releasable engagement, and other connections.

First end **42** also includes a first hollow needle cannula **52** having an internal lumen **54** extending therethrough. First needle cannula **52** extends away from coupler **40** at first end **42** along a common axis with passageway **46.** When first end **42** engages first sampling tube holder **12,** first needle cannula **52** extends into first sampling tube holder **12** through opening **30.** First needle cannula **52** further includes a sharp puncture tip **56** typically provided for puncturing a stopper or septum of a collection tube when first end **42** of coupler **40** engages first sampling tube holder **12.** Desirably, a piercable elastomeric sleeve or sheath **58** extends from first end **42** and about the end of first needle cannula **52** thereby encompassing the puncture tip **56.** As will be discussed in more detail herein in terms of use of the fluid transfer device, this sleeve or sheath **58** acts as a valve for preventing the contents of the sampling tubes from being expelled. In addition, sleeve or sheath **58** functions as a safety mechanism for protection from puncture tip **56.**

Likewise, second end **44** includes a second hollow needle cannula **152** having an internal lumen **154** extending therethrough. Second needle cannula **152** extends away from coupler **40** at second end **44** along a common axis with passageway **46.** When second end **44** engages second sampling tube holder **112,** second needle cannula **152** extends into second sampling tube holder **112** through opening **130.** Second needle cannula **152** further includes a sharp puncture tip **156** in a similar manner as first needle cannula **52,** which is provided for puncturing a stopper or septum of a collection tube when second end **42** of coupler **40** engages second sampling tube holder **112.** As with first needle cannula 52, a piercable elastomeric sleeve or sheath **158** may extend from second end **42** of coupler **40** and about the end of second needle cannula **152** thereby encompassing the puncture tip **156.** Sleeve or sheath **158** acts as a valve as well as a safety mechanism for protection from puncture tip **156** in a similar manner as discussed above.

First needle cannula **52** and second needle cannula **152** may be provided as distinct and separate members, so long as fluid flow is established between the respective lumen, for example, via passageway **46** of coupler **40.** Alternatively, first needle cannula **52** and second needle cannula **152** may be provided as a single needle cannula extending through passageway **46** of coupler **40,** with first puncture tip **56** and second puncture tip **156** extending respectively from first end **42** and second end **44** of coupler **40.**

First needle cannula **52** and second needle cannula **152** may be attached to coupler **40** by mechanical engagement or through adhesive means. Regardless of the attachment mechanism, the force required for first needle cannula **52** and/or second needle cannula **152** to pierce a stopper of a blood container or collection tube is less than the force required to unattach first needle cannula **52** and/or second needle cannula **152** from coupler **40.**

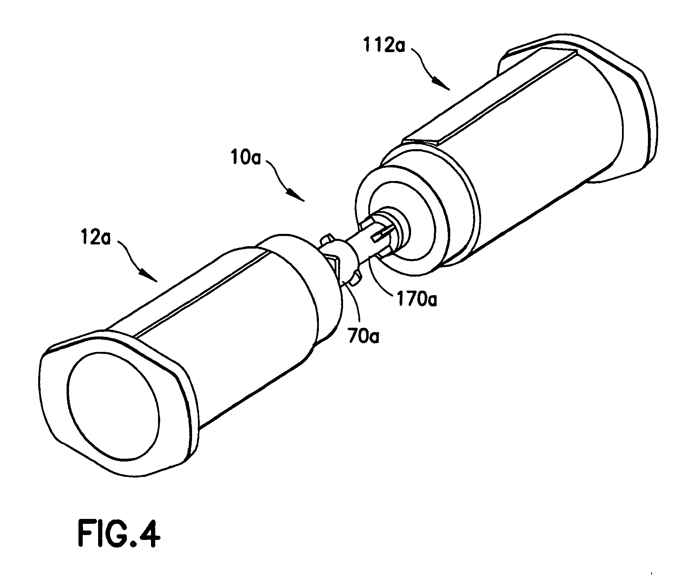

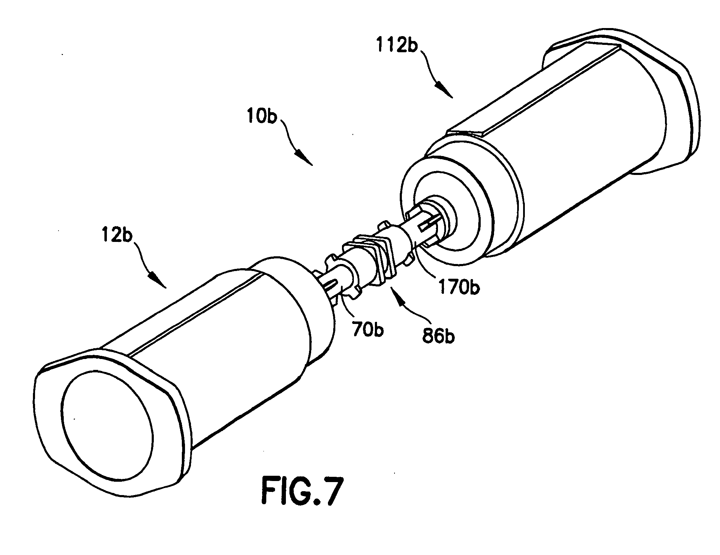

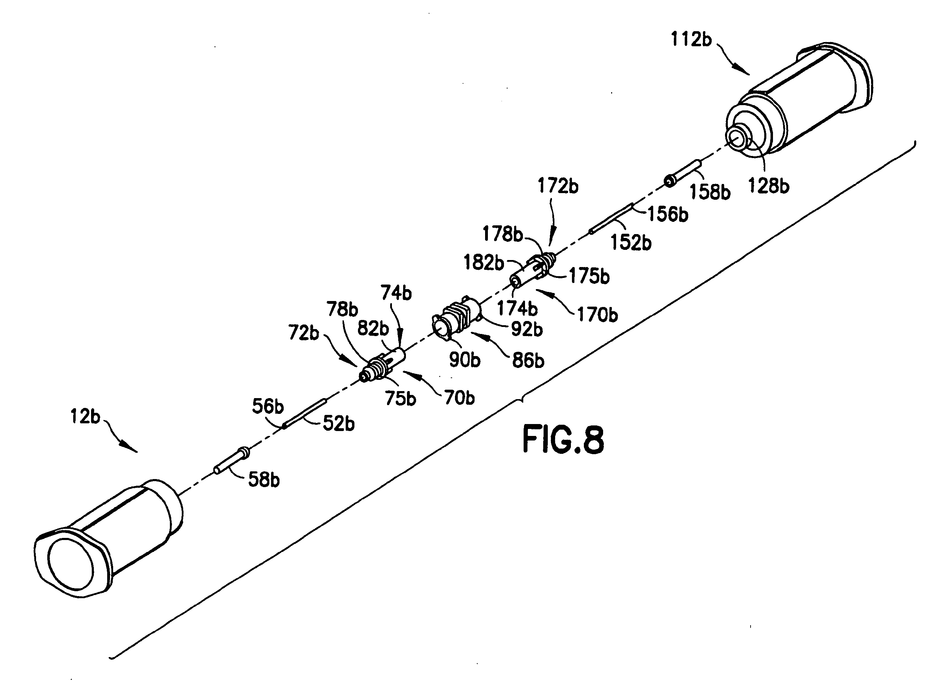

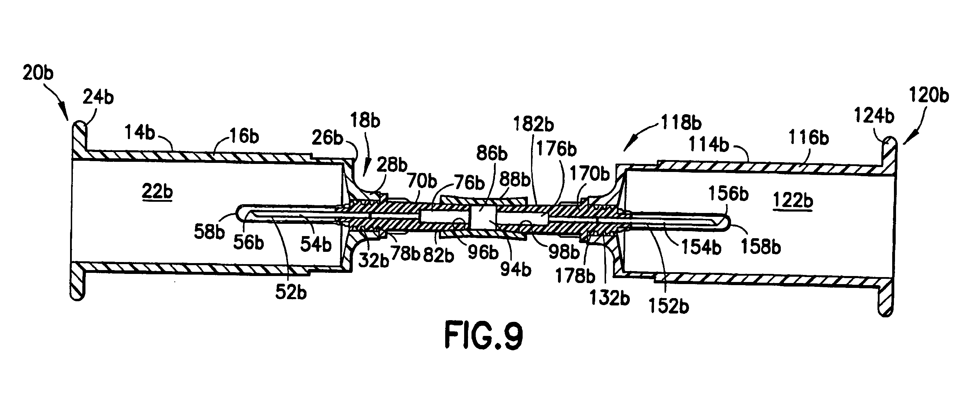

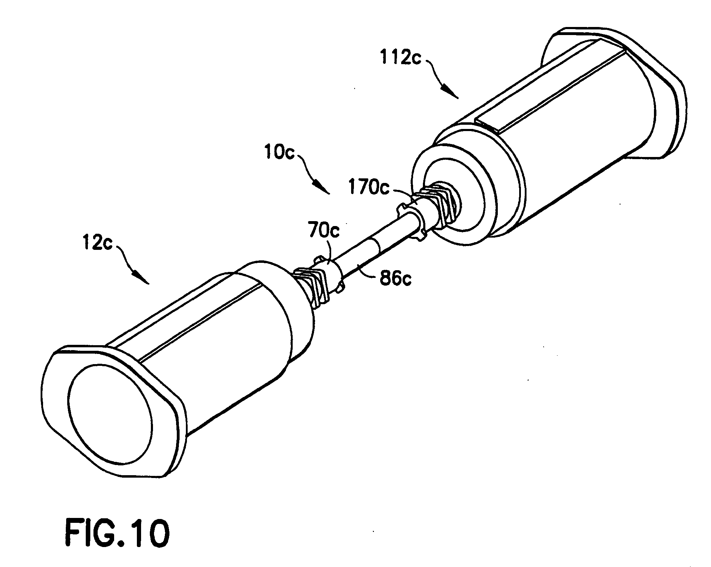

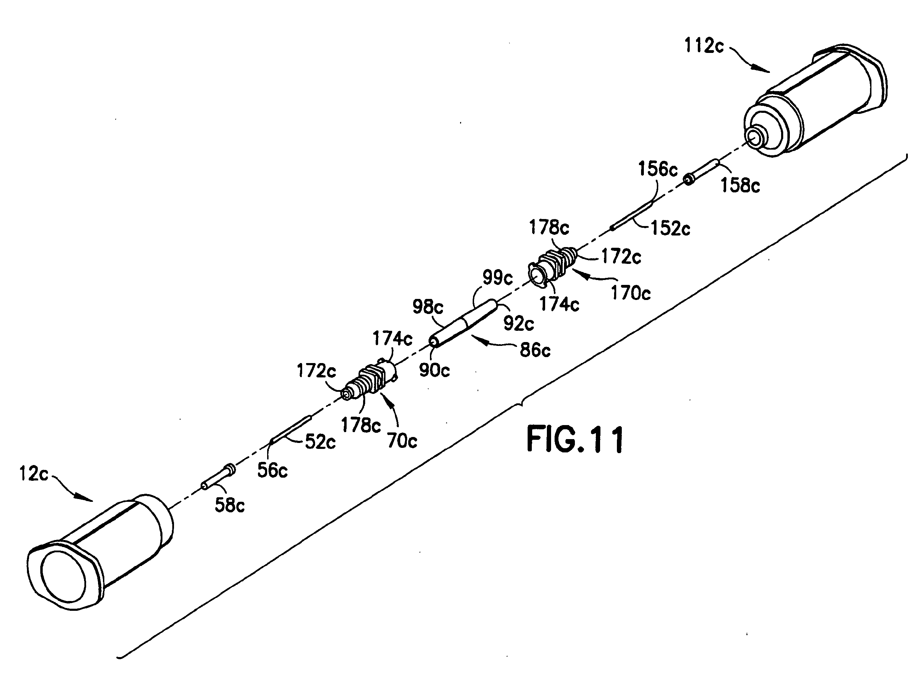

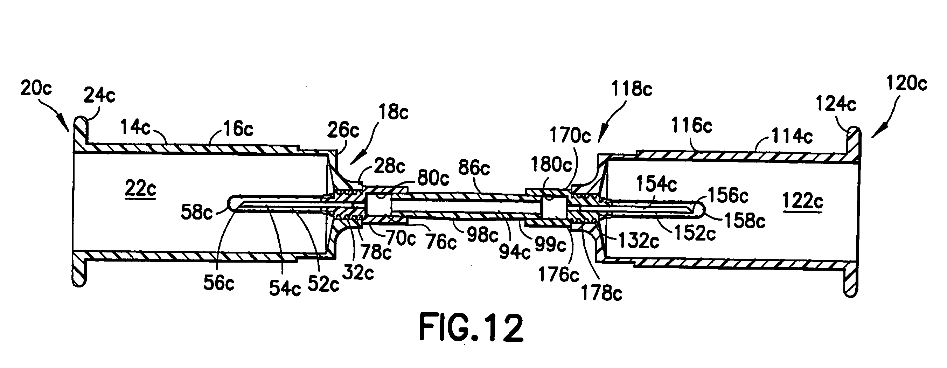

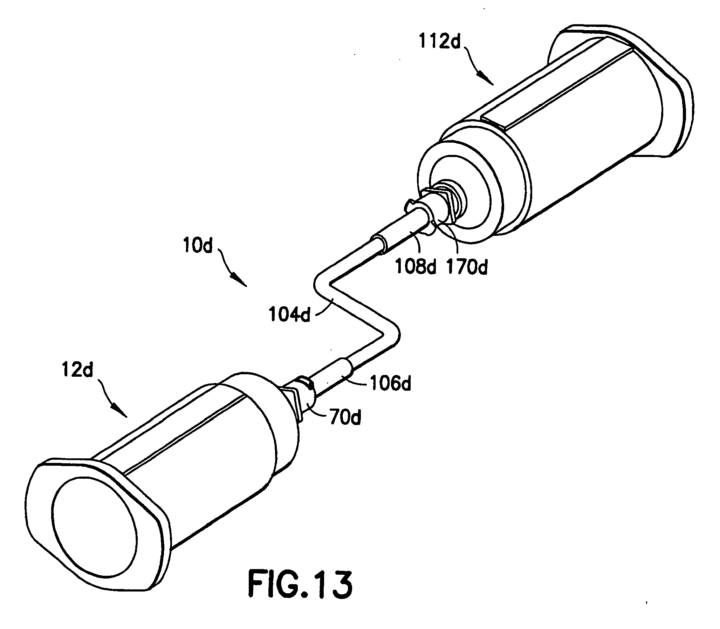

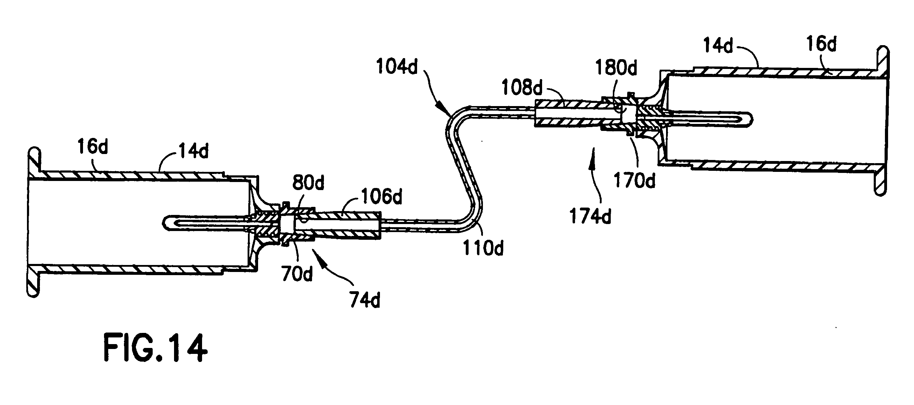

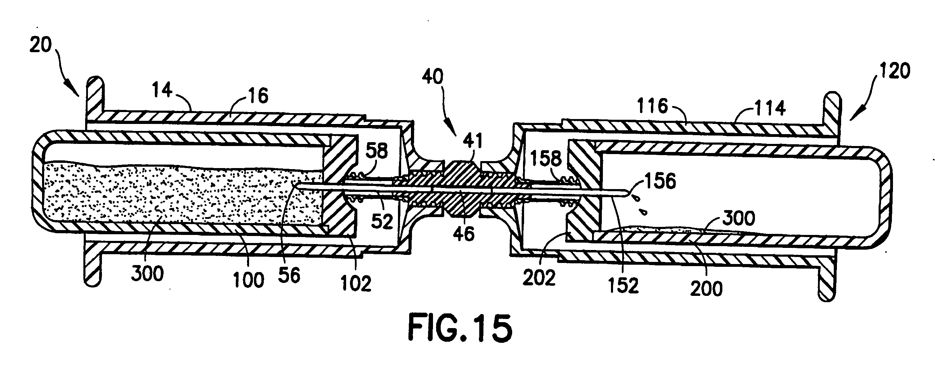

FIGS. 4-15 depict further configurations which include many components that are substantially identical to the components of FIGS. 1-3. Accordingly, similar components performing similar functions will be numbered identically to those components of FIGS. 1-3, except that a suffix "a" will be used to identify those similar components in FIGS. 4-6, a suffix "b" will be used to identify those similar components in FIGS. 7-9, a suffix "c" will be used to identify those similar components in FIGS. 10-12, and a suffix "d" will be used to identify those similar components in FIGS. 13-15.

FIGS. 4-6 illustrate a fluid transfer device **10a** which does not fall within the scope of the claims, where the coupler **40a** includes a first hub **70a** and a second hub **170a.** First hub 70a includes holder end **72a** for engagement with first sampling tube holder **12a,** and a connector end **74a** for mating with second hub **170a.** A flange **75a** may be provided between holder end **72a** and connector end **74a.** An internal passageway **76a** extends through first hub **70a** between holder end **72a** and connector end **74a.** Holder end **72a** further includes structure for mating with first sampling tube holder **12a,** such as external threads **78a.**

Second hub **170a** includes holder end **172a** for engagement with second sampling tube holder **112a,** and connector end **174a** for mating with first hub **70a,** with flange **175a** provided between holder end **172a** and connector end **174a.** An internal passageway **176a** extends through second hub **170a** between holder end **172a** and connector end **174a** in a similar manner as with first hub **70a.** Holder end **172a** further includes structure for mating with second sampling tube holder **112a,** such as external threads **178a.**

In such a configuration in which the coupler is provided as two separate components in the form of first hub **70a** and second hub **170a,** first needle cannula **52a** is associated with first hub **70a** and second needle cannula **152a** is associated with second hub **170a.** As such, first needle cannula **52a** extends from holder end **72a** of first hub **70a,** for extension through opening **30a** and within internal opening **22a** of first holder **12a** when first hub **70a** is mated with first holder **12a.** Likewise, second needle cannula **152a** extends from holder end **172a** of second hub **170a,** for extension through opening **130a** and within internal opening **122a** of second holder **112a** when second hub **170a** is mated with second holder **112a.** Elastomeric sleeves or sheaths **58a** and **158a** extend from respective ends of first and second hubs **70a, 170a** to encompass the respective first and second puncture tips **56a, 156a,** in a similar manner as discussed above.

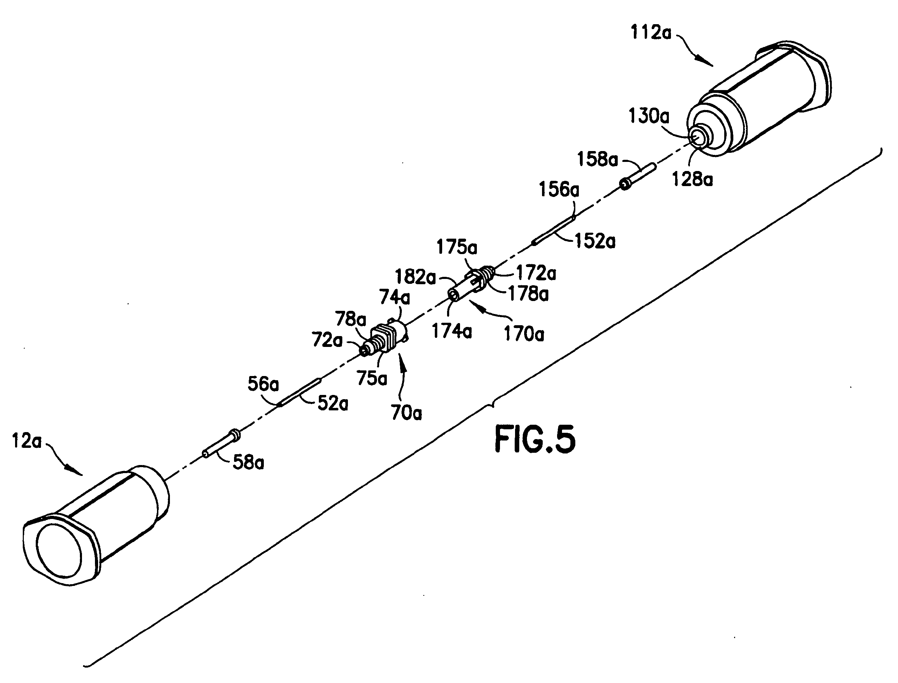

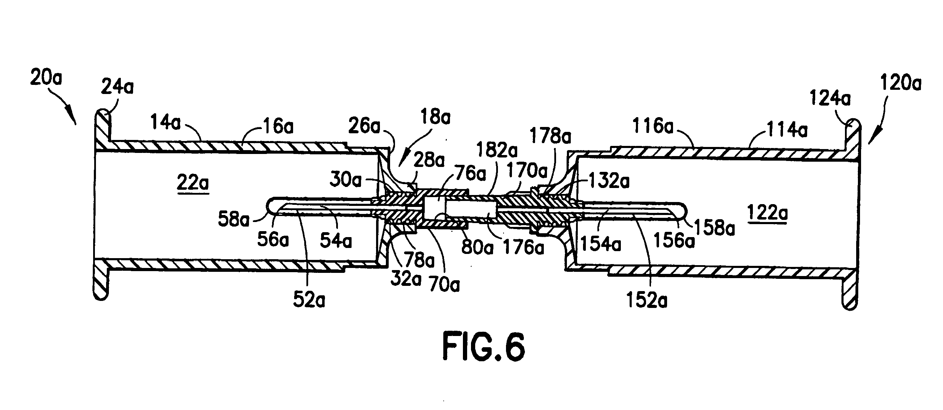

Desirably, connector end **74a** of first hub **70a** and connector end **174a** of second hub **170a** include structure for mating engagement with each other. For example, one of first hub **70a** or second hub **170a** may include a male taper surface while the other may include a female taper surface, for establishing a luer-type engagement therebetween. In particular, as shown in FIGS. 5 and 6, first hub **70a** may be provided with a female taper surface **80a,** while second hub **170a** is provided with a male taper surface **182a.** Female taper surface **80a** is capable of accommodating male taper surface **182a** therein, thereby providing structure for mating engagement between first hub **70a** and second hub **170a.** Female taper surface **80a** and male taper surface **182a** are engageable, for example, in a friction fit to attach first hub **70a** and second hub **170a** in a frictional engagement therebetween. Alternatively or in addition to the frictional engagement, an adhesive may be provided between female taper surface **80a** and male taper surface **182a.**

FIGS. 7-9 illustrate a fluid transfer device **10b** in a further configuration which does not fall within the scope of the claims in which coupler **40b** includes a first hub **70b** and a second hub **170b** in a similar manner as in the configuration of FIGS. 4-6, but with the respective connector ends **74b, 174b** both including a male taper surface **82b, 182b,** respectively. As such, coupler **40b** in FIGS. 7-9 further includes a connector 86b for establishing connection between first hub **70b** and second hub **170b.** In particular, connector **86b** is defined by a tubular wall **88b** extending between a first end **90b** and a second end **92b,** with a passageway **94b** extending therethrough. First end **90b** of connector **86b** includes a female taper surface **96b,** and second end **92b** of connector **86b** also includes a female taper surface **97b.** As such, connector **86b** as shown in FIGS. 7-9 provides for a male-to-male connection, such as for connecting male taper surface **82b** of first hub **70b** and male taper surface **182b** of second hub **170b,** thereby establishing fluid flow therebetween through passageway **94b.**

FIGS. 10-12 illustrate a further configuration which does not fall within the scope of the claims of a fluid transfer device **10c** which is similar to the embodiment depicted in FIGS. 7-9, but with the coupler including a female-to-female type connector instead of the male-to-male type connector of FIGS. 7-9. In particular, coupler **40c** includes a first hub **70c** and a second hub **170c** in a similar manner as described above, but with the respective connector ends **74c, 174c** both including a female taper surface **80c, 180c,** respectively. Coupler **40c** in FIGS. 10-12 therefore further includes a connector 86c for establishing connection between first hub **70c** and second hub **170c.** Connector **86c** is defined by a tubular wall **88c** extending between a first end **90c** and a second end **92c,** with a passageway **94c** extending therethrough. First end **90c** of connector **86c** includes a male taper surface **98c,** and second end **92c** of connector **86c** also includes a male taper surface **99c.** As such, connector **86c** as shown in FIGS. 10-12 provides for a female-to-female connection, such as for connecting female taper surface **80c** of first hub **70c** and female taper surface **180c** of second hub **170c,** thereby establishing fluid flow therebetween through passageway **94c.**

FIGS. 13-14 depict an embodiment of a fluid transfer device 10d according to the invention, which is similar to the previous embodiments, but with the coupler including a flexible tubing extending between first hub **70d** and second hub **170d,** instead of a connector. Flexible tubing **104d** extends between a first end **106d** and a second end **108d,** with an internal passageway **110d** extending therethrough. First end **106d** is attached to connector end **74d** of first hub **70d,** while second end **108d** is attached to connector end **174d** of second hub **170d.** Desirably, connector ends **74d, 174d,** of first and second hubs **70d, 170d,** are female taper surfaces **80d** and **180d,** respectively, thereby providing a tapering surface for engagement with the respective ends of flexible tubing **104d.** Flexible tubing **104d** provides for fluid passage between first hub **70d** and second hub **170d.**

The present invention further encompasses a method of assembling a fluid transfer device as well as a method of transferring fluids using a fluid transfer device as defined in claims 11 and 13. Accordingly, a fluid transfer device according to the invention may be provided for use in a pre-packaged and pre-sterilized form. Desirably, the first sampling tube holder and the second sampling tube holder of the forms described above are mechanically or chemically bonded to the coupler, for example by sonically welding or adhesively attaching the first sampling tube holder to the holder end of a first hub and the second sampling tube holder to the holder end of a second hub. As such, the first sampling tube holder can be provided with the first hub attached thereto, and the second sampling tube holder can be provided with the second hub attached thereto. The first and second sampling tube holders can thereafter be mated and joined through the first and second hubs, such as through frictional engagement between the respective connector surfaces, or by mating and sonically welding or adhesively attaching the connector ends of the first and second hubs. Moreover, a connector may be provided between the connector ends of the first and second hubs, as discussed with respect to the alternate configurations described above.

Desirably, the assembly is assembled, packaged and sterilized as a single unit, to provide an assembly for use as a fluid transfer device. It is further contemplated that the components can be assembled in sub-assemblies, such as with the first hub attached to the first sampling tube holder and the second hub attached to the second sampling holder, with the sub-assemblies being joined together just prior to use, such as by frictional engagement therebetween or through a connector.

In use, the fluid transfer assembly is provided as described with respect to any of the above-referenced configurations, although use of the invention will be described generally in terms of the configuration of FIGS. 1-3 and 15, with first sampling tube holder **12** attached to second sampling tube holder **112** through coupler **40.** With fluid transfer device **10** provided as such, two separate sampling tubes **100, 200,** each having a puncturable closure or stopper **102, 202,** are provided for transfer of a fluid therebetween. One of the sampling tubes contains a fluid **300** such as blood therein, for transfer to the other sampling tube. The first sampling tube **100** containing the sample fluid **300** therein is inserted into internal opening **22** of first sampling tube holder **12** through rearward end **20** thereof, while the second sampling tube **200** is inserted into internal opening **122** of second sampling tube holder **112** through rearward end **120** thereof.

With fluid transfer device **10** assembled, first puncture tip **56** of first needle cannula **52** extends within internal opening **22** of first sampling tube holder **12,** and second puncture tip **156** of second needle cannula **152** extends within internal opening **122** of second sampling tube holder **112.** The first sampling tube **100** is inserted into first sampling tube holder **12** to cause the stopper **102** closing the end of the first sampling tube **100** to contact and displace elastomeric sleeve or sheath **58.** Further insertion of the first sampling tube **100** within first sampling tube holder **12** causes first puncture tip **56** to puncture the stopper **102** and causes first needle cannula **52** to be inserted into the interior of the first sampling tube **100** in fluid engagement with the sample fluid **300** present therein. At this point, the elastomeric sleeve or sheath **158** which extends about second needle cannula **152** acts as a valve, preventing the convents of first sampling tube **100** from being expelled through passageway **46** and into the ambient environment. The stopper **202** of second sampling tube **200** is then punctured by second puncture tip **156** in a similar manner through displacement of sheath **158** and insertion of second puncture tip **156** through stopper **202,** such that second needle cannula **152** is in fluid engagement with the interior of the second sampling tube **200.** Since first needle cannula **52** and second needle cannula **152** are in fluid communication therebetween, such as through passageway **46** of coupler **40,** and since first needle cannula **52** now extends within the first sampling tube **100** and since second needle cannula **152** now extends within the second sampling tube **200,** a path for fluid flow is established between the first and second sampling tubes **100, 200** through fluid transfer device **10.**

Desirably, the second sampling tube **200** into which the fluid sample is to be transferred is evacuated at a pressure below that of the first sampling tube **100** containing the sample fluid **300** to be transferred. As such, the negative pressure within the second sampling tube **200** causes fluid flow from the first sampling tube **100,** through first and second cannulas **52** and **152,** and into the second sampling tube **200** automatically upon insertion of second needle cannula **152** into the second sampling tube **200.**

The amount of fluid transferred between the tubes can be controlled by the length of time of transfer or by the amount of vacuum in the second sampling tube. It is contemplated that the pressure within the second sampling tube can be fixed at a specific value in order to transfer a predetermined quantity of fluid from the first sampling tube into the second sampling tube. As such, the exact pressure value will be dependent upon the amount and type of fluid being transferred, and, thus, can be controlled during preparation of the empty sampling tubes, i.e., during manufacturing.

Upon transfer of the desired amount of fluid, the first and second sampling tubes can be removed from the respective first and second sampling tube holders **12, 112** and stored for appropriate analytical procedures. Fluid transfer device **10** can then be appropriately discarded.

By providing the fluid transfer device as described herein, fluids can be safely and conveniently transferred between sample containers, and in particular between evacuated sample tubes. Moreover, in many instances it is desirable to draw a partial sample from a sample after it has been collected. For example, in certain instances it is desirable to collect a blood sample and to allow the sample to begin to coagulate, and to then sample a portion of that blood sample for further analysis. The present invention provides an effective mechanism and method for this particular procedure, without requiring transfer between a secondary container or syringe, and without risk of contamination through a pouring off procedure.

## Claims

1. A fluid transfer device comprising:
a first sampling tube holder (12d) having an internal opening (22) capable of accommodating a first sampling tube therein;
a second sampling tube holder (112d) having an internal opening capable of accommodating a second sampling tube therein; and
a coupler comprising a first hub (70d), having a holder end, connector end, and a first cannula extending from said holder end and a second hub (170d) having a holder end, a connector end, and a second cannula extending from said holder end, and a connector being flexible tube-like structure (104d, 108d, 106d) and having a first end and a second end, said first end of said connector in fluid engagement with said connector end of said first hub and said second end of said connector in fluid engagement with said connector end of said second hub, for joining said first sampling tube holder (12d) and said second sampling tube holder (112d), said coupler including an internal fluid path extending between a first end and a second end, said first end including a first puncture tip extending into said internal opening of said first sampling tube holder (12d) and said second end including a second puncture tip extending into said internal opening of said second sampling tube holder (112d),
wherein said fluid transfer device is capable of establishing a path for fluid flow between a first sampling tube accommodated within said internal opening of said first sampling tube holder (12d) and a second sampling tube accommodated within said internal opening of said second sampling tube holder (112d) through said coupler.

2. The fluid transfer device according to claim 1, wherein said first and second cannulae are attached within an internal passageway of said first and second hubs (70d, 170d) by mechanical or adhesive means.

3. The fluid transfer device according to claim 1, wherein said holder end of said first hub includes structure for engagement with said first sampling tube holder and said holder end of said second hub includes structure for engagement with said second sampling tube holder.

4. The fluid transfer device according to claim 3, wherein:
said first sampling tube holder (12d) includes internal threads and said first end of said first hub (70d) includes external threads for engagement with said internal threads of said first sampling tube holder, and
said second (112d) sampling tube holder includes internal threads and said second end of said second hub (170d) includes external threads for engagement with said internal threads of said second sampling tube holder (112d).

5. The fluid transfer device according to claim 3, wherein said holder end of said first hub (70d) is attached to said first sampling tube holder (12d) such that the force required for said first end of said cannula to pierce a stopper of a first sampling tube accommodated within said internal opening of said first sampling tube holder (12d) is less than the force required to unattach said first end of said first hub (70d) from said first sampling tube holder (12d), and said holder end of said second hub (170d) is attached to said second sampling tube holder (112d) such that the force required for said holder end of said cannula to pierce a stopper of a second sampling tube accommodated within said internal opening of said second sampling tube holder (112d) is less than the force required to unattach said holder end of said hub (170d) from said second sampling tube holder (112d).

6. The fluid transfer device according to claim 3, wherein said holder end of said first hub (70d) is adhesively attached to said first sampling tube holder (12d) and said holder end of said second hub (170d) is adhesively attached to said second sampling tube holder (112d).

7. The fluid transfer device according to claim 1, further comprising:
a first elastomeric sleeve extending about a first end of said first cannula and within said internal opening of said first sampling tube holder (12d); and
a second elastomeric sleeve extending about a second end of said second cannula and within said internal opening of said second sampling tube holder (112d).

8. The fluid transfer device according to claim 1, wherein said first sampling tube holder (12d) and said second sampling tube holder (112d) are sized so as to accommodate respective first and second sampling tubes having a maximum diameter of about 16 millimeters.

9. The fluid transfer device according to claim 1, wherein:
said first end (70d) of said hub includes external threads configured to engage internal threads of the first sampling tube holder (12d), and
said second end (170d) of said hub includes external threads configured to engage internal threads of the second sampling tube holder (112d).

10. The fluid transfer device according to claim 1, further comprising:
a piercable sleeve extending about said first puncture tip a piercable sleeve extending about said second puncture tip.

11. A method of transferring body fluids between a first sampling tube and a second sampling tube comprising the following steps:
a) providing a fluid transfer device comprising first and second sampling tube holders (12d, 112d) for accommodating first and second sampling tubes therein, said first and second sampling tube holders (12d, 112d) being joined together through a coupler comprising a first hub (70d), having a holder end, connector end, and a first cannula extending from said holder end and a second hub (170d) having a holder end, a connector end, and a second cannula extending from said holder end, and a connector having a first end and a second end, being a flexible tube-like structure, said first end of said connector in fluid engagement with said connector end of said first hub and said second end of said connector in fluid engagement with said connector end of said second hub, the first cannula having a first puncture tip and the second canula having a second puncture tip such that said first puncture tip extends into said first sampling tube holder (12d) and said second puncture tip extends into said second sampling tube holder (112d), said second puncture tip including a piercable sleeve extending thereabout, and providing said first and second sampling tube holders (12d, 112d) for accommodating first and second sampling tubes therein, and
joining said first and second sampling tube holders (12d, 112d) through said coupler such that said first puncture tip of said first cannula extends into said first sampling tube holder (12d) and said second puncture tip of said second cannula extends into said second sampling tube holder (112d).;
b) inserting a first sampling tube having a puncturable closure within said first sampling tube holder (12d) and puncturing said closure of said first sampling tube with said first puncture tip within said first sampling tube holder (12d) ; and
c) inserting a second sampling tube having a puncturable closure within said second sampling tube holder (112d) and puncturing said closure of said second sampling tube with said second puncture tip within said second sampling tube holder (112d);
wherein fluid flow is established between said first sampling tube and said second sampling tube through said cannula, thereby transferring fluid between said first sampling tube and said second sampling tube.

12. The method of claim 11, wherein first and second puncture tips include a piercable sleeve extending thereabout.

13. A method of assembling a fluid transfer device comprising the following steps:
a) providing first and second sampling tube holders (12d, 112d) for accommodating first and second sampling tubes therein; and
b) joining said first and second sampling tube holders (12d, 112d) through a coupler,
wherein said coupler comprises a first hub (70d), having a holder end, connector end, and a first cannula extending from said holder end, said first cannula having a first puncture tip with a first piercable sleeve extending thereabout and a second hub (170d) having a holder end, a connector end, and a second cannula extending from said holder end, said second cannula having, a second puncture tip with a second piercable sleeve extending thereabout, and a connector having a first end and a second end, being a flexible tube-like structure, said first end of said connector in fluid engagement with said connector end of said first hub and said second end of said connector in fluid engagement with said connector end of said second hub,and wherein said step a) comprises providing said first sampling tube holder (12d) joined with said first hub (70d) with said first puncture tip extending within said first sampling tube holder (12d) and providing said second sampling tube holder (112d) joined with said second hub (170d) with said second puncture tip extending within said second sampling tube holder (112d); and said step b) comprises mating said first hub (70d) with said second hub (170d) to join said first and second sampling tube holders (12d, 112d).

14. The method of claim 13, further comprising:
c) packaging said first and second sampling tube holders (12d, 112d) joined through said coupler.

15. The method of claim 14, further comprising:
d) sterilizing said first and second sampling tube holders (12d, 112d) joined through said coupler.

## Patentansprüche

1. Eine Flüssigkeitsübertragungsvorrichtung, enthaltend:
- einen ersten Halter (12d) für Probeentnahmeröhrchen, der eine innere Öffnung (22) hat, die darin ein erstes Probeentnahmeröhrchen aufnehmen kann;
- einen zweiten Halter (112d) für Probeentnahmeröhrchen, der eine innere Öffnung hat, die darin ein zweites Probeentnahme-röhrchen aufnehmen kann; und
- eine Kupplung, die eine erste Buchse (70d), die ein Halterende, ein Konnektorende und eine erste Kanüle aufweist, die sich von dem Halterende erstreckt, und eine zweite Buche (170d), die ein Halterende, ein Konnektorende und eine zweite Kanüle hat, die sich von dem Halterende erstreckt, und einen Konnektor aufweist, der eine flexible Röhrchen-ähnliche Struktur (104d, 108d, 106d) ist und ein erstes Ende und ein zweites Ende hat, wobei das erste Ende des Konnektors in Flüssigkeitseingriff mit dem Konnektorende der ersten Buchse und das zweite Ende des Konnektors in Flüssigkeitseingriff mit dem Konnektorende der zweiten Buchse steht, um den ersten Halter (12d) für Probeentnahmeröhrchen mit dem zweiten Halter (112d) für Probeentnahmeröhrchen zu verbinden, wobei die Kupplung eine innere Flüssigkeitsbahn enthält, die sich zwischen einem ersten Ende und einem zweiten Ende erstreckt, wobei das erste Ende eine erste Einstichspitze enthält, die sich in die innere Öffnung des ersten Halters (12d) für Probeentnahmeröhrchen erstreckt, und das zweite Ende eine zweite Einstichspitze enthält, die sich in die innere Öffnung des zweiten Halters (112d) für Probeentnahmeröhrchen erstreckt,
wobei die Flüssigkeitsübertragungsvorrichtung in der Lage ist, eine Bahn für einen Flüssigkeitsstrom zwischen einem ersten Probeentnahme-röhrchen, das in die innere Öffnung des ersten Halters (12d) für Probeentnahmeröhrchen aufgenommen ist, und einem zweiten Probeentnahmeröhrchen, das in die innere Öffnung des zweiten Halters (112d) für Probeentnahmeröhrchen aufgenommen ist, durch die Kupplung hindurch herzustellen.

2. Die Flüssigkeitsübertragungsvorrichtung nach Anspruch 1, wobei die erste und die zweite Kanüle in einem inneren Kanal der ersten und der zweiten Buchse (70d, 170d) durch mechanische Mittel oder Klebemittel befestigt sind.

3. Die Flüssigkeitsübertragungsvorrichtung nach Anspruch 1, wobei das Halterende der ersten Buchse eine Struktur zum Eingriff mit dem ersten Halter für Probeentnahmeröhrchen hat und das Halterende der zweiten Buchse eine Struktur zum Eingriff mit dem zweiten Halter für Probeentnahmeröhrchen hat.

4. Die Flüssigkeitsübertragungsvorrichtung nach Anspruch 3, wobei:
- der erste Halter (12d) für Probeentnahmeröhrchen ein Innengewinde und das erste Ende der ersten Buchse (70d) ein Außengewinde zum Eingriff mit dem Innengewinde des ersten Halters für Probeentnahmeröhrchen hat, und
- der zweite Halter (112d) für Probeentnahmeröhrchen ein Innengewinde und das zweite Ende der zweiten Buchse (170d) ein Außengewinde zum Eingriff mit dem Innengewinde des zweiten Halters (112d) für Probeentnahmeröhrchen hat.

5. Die Flüssigkeitsübertragungsvorrichtung nach Anspruch 3,
wobei das Halterende der ersten Buchse (70d) an dem ersten Halter (12d) für Probeentnahmeröhrchen so befestigt ist, dass die Kraft, die erforderlich ist, damit das erste Ende der Kanüle einen Stopfen eines ersten Probeentnahmeröhrchens durchsticht, das in die innere Öffnung des ersten Halters (12d) für Probeentnahmeröhrchen aufgenommen ist, kleiner ist als die Kraft, die erforderlich ist, um das erste Ende der ersten Buchse (70d) von dem ersten Halter (12d) für Probeentnahmeröhrchen zu lösen, und dass das Halterende der zweiten Buchse (170d) an dem zweiten Halter (112d) für Probeentnahmeröhrchen so befestigt ist, dass die Kraft, die erforderlich ist, damit das Halterende der Kanüle einen Stopfen eines zweiten Probeentnahmeröhrchens durchsticht, das in die innere Öffnung des zweiten Halters (112d) für Probeentnahmeröhrchen aufgenommen ist, kleiner ist als die Kraft, die erforderlich ist, um das Halterende der Buchse (170d) von dem zweiten Halter (112d) für Probeentnahmeröhrchen zu lösen.

6. Die Flüssigkeitsübertragungsvorrichtung nach Anspruch 3,
wobei das Halterende der ersten Buchse (70d) klebend an dem ersten Halter (12d) für Probeentnahmeröhrchen befestigt ist und wobei das Halterende der zweiten Buchse (170d) klebend an dem zweiten Halter (112d) für Probeentnahmeröhrchen befestigt ist.

7. Die Flüssigkeitsübertragungsvorrichtung nach Anspruch 1, ferner enthaltend:
- eine erste elastomere Hülse, die sich um ein erstes Ende der ersten Kanüle und in der inneren Öffnung des ersten Halters (12d) für Probeentnahmeröhrchen erstreckt;
- eine zweite elastomere Hülse, die sich um ein zweites Ende der zweiten Kanüle und innerhalb der inneren Öffnung des zweiten Halters (112d) für Probeentnahmeröhrchen erstreckt.

8. Die Flüssigkeitsübertragungsvorrichtung nach Anspruch 1,
wobei der erste Halter (12d) für Probeentnahmeröhrchen und der zweite Halter (112d) für Probeentnahmeröhrchen so bemessen sind, dass sie erste und zweite Probeentnahmeröhrchen mit einem maximalen Durchmesser von etwa 16 mm aufnehmen.

9. Die Flüssigkeitsübertragungsvorrichtung nach Anspruch 1, wobei:
- das erste Ende (70d) der Buchse ein Außengewinde enthält, das so ausgebildet ist, dass es in das Innengewinde des ersten Halters (12d) für Probeentnahmeröhrchen eingreift, und
- das zweite Ende (170d) der Buchse ein Außengewinde enthält, das so ausgebildet ist, dass es in das Innengewinde des zweiten Halters (112d) für Probeentnahmeröhrchen eingreift.

10. Die Flüssigkeitsübertragungsvorrichtung nach Anspruch 1, ferner enthaltend:
- eine durchstechbare Hülse, die sich um die erste Einstichspitze erstreckt,
- eine durchstechbare Hülse, die sich um die zweite Einstichspitze erstreckt.

11. Ein Verfahren zum Übertragen von Körperflüssigkeiten zwischen einem ersten Probeentnahmeröhrchen und einem zweiten Probeentnahmeröhrchen, enthaltend die folgenden Schritte:
a) Bereitstellen einer Flüssigkeitsübertragungsvorrichtung, die einen ersten und einen zweiten Halter (12d, 112d) für Probeent-nahmeröhrchen zur Aufnahme eines ersten und eines zweiten Probeentnahmeröhrchens darin hat, wobei der erste und der zweite Halter (12d, 112d) für Probeentnahmeröhrchen durch eine Kupplung miteinander verbunden sind, die eine erste Buchse (70d), die ein Halterende, ein Konnektorende und eine erste Kanüle hat, die sich von dem Halterende erstreckt, und eine zweite Buchse (170d), die ein Halterende, ein Konnektorende und eine zweite Kanüle hat, die sich von dem Halterende erstreckt, und einen Konnektor aufweist, der ein erstes Ende und ein zweites Ende hat und eine flexible röhrchenähnliche Struktur ist, wobei das erste Ende des Konnektors in Fluideingriff mit dem Konnektorende der ersten Buchse und das zweite Ende des Konnektors in Fluideingriff mit dem Konnektorende der zweiten Buchse steht, wobei die erste Kanüle eine erste Einstichspitze und die zweite Kanüle eine zweite Einstichspitze haben, so dass die erste Einstichspitze sich in den ersten Halter (12d) für Probeentnahmeröhrchen und die zweite Einstichspitze sich in den zweiten Halter (112d) für Probeentnahmeröhrchen erstreckt, wobei die zweite Einstichspitze eine durchstechbare Hülse aufweist, die sich darum erstreckt, und Bereitstellen des ersten und des zweiten Halters (12d, 112d) für Probeentnahmeröhrchen zum Aufnehmen eines ersten und eines zweiten Probeentnahmeröhrchens darin und Verbinden des ersten und zweiten Halters (12d, 112d) für Probeentnahmeröhrchen durch die Kupplung, derart, dass sich die erste Einstechspitze der ersten Kanüle in den ersten Halter (12d) für Probeentnahmeröhrchen erstreckt und dass sich die zweite Einstechspitze der zweiten Kanüle in den zweiten Halter (112d) für Probeentnahmeröhrchen erstreckt;
b) Einsetzen eines ersten Probeentnahmeröhrchens, das einen einstechbaren Verschluss enthält, in den ersten Halter (12d) für Probeentnahmeröhrchen und Durchstechen des Verschlusses des ersten Probeentnahmeröhrchens mit der ersten Einstichspitze innerhalb des ersten Halters (12d) für Probeentnahmeröhrchen; und
c) Einsetzen eines zweiten Probeentnahmeröhrchens mit einem durchstechbaren Verschluss in den zweiten Halter (112d) für Probeentnahmeröhrchen und Durchstechen des Verschlusses des zweiten Probeentnahmeröhrchens mit der zweiten Einsteckspitze innerhalb des zweiten Halters (112d) für Probeentnahmeröhrchen; wobei ein Flüssigkeitsstrom zwischen dem ersten Probeentnahme-röhrchen und dem zweiten Probeentnahmeröhrchen durch die Kanüle hergestellt und hierdurch Flüssigkeit zwischen dem ersten Probeentnahmeröhrchen und dem zweiten Probeentnahmeröhrchen übertragen wird.

12. Das Verfahren nach Anspruch 11, wobei die erste und die zweite Einstechspitze eine durchstechbare Hülse enthalten, die sich darum erstreckt.

13. Ein Verfahren zum Zusammensetzen einer
Flüssigkeitsübertragungs-vorrichtung, enthaltend die folgenden Schritte:
a) Bereitstellen eines ersten und eines zweiten Halters (12d, 112d,) für Probeentnahmeröhrchen zur Aufnahme eines ersten und eines zweiten Probeentnahmeröhrchens darin; und
b) Verbinden des ersten und des zweiten Halters (12d, 112d) für Probeentnahmeröhrchen durch eine Kupplung;
wobei die Kupplung eine erste Buchse (70d), die ein Halterende, ein Konnektorende und eine erste Kanüle enthält, die sich von dem Halterende erstreckt, wobei die erste Kanüle eine erste Einstechspitze mit einer ersten durchstechbaren Hülse, die sich darum erstreckt, aufweist, und eine zweite Buchse (170d), die ein Halterende, ein Konnektorende und eine zweite Kanüle hat, die sich von dem Halterende erstreckt, wobei die zweite Kanüle eine zweite Einstechspitze mit einer zweiten durchstechbaren Hülse, die sich darum erstreckt, und einen Konnektor aufweist, der ein erstes Ende und ein zweites Ende hat und eine flexible, Röhrchen-ähnliche Struktur ist, wobei das erste Ende des Konnektors in Flüssigkeitseingriff mit dem Konnektorende der ersten Buchse und das zweite Ende des Konnektors in Flüssigkeitseingriff mit dem Konnektorende der zweiten Buchse steht, und wobei der Schritt a) einschließt, dass der erste Halter (12d) für Probeentnahmeröhrchen mit der ersten Buchse (70d) verbunden ist, wobei sich die erste Einstichspitze innerhalb des ersten Halters (12d) für Probeentnahmeröhrchen erstreckt, und der zweite Halter (112d) für Probeentnahmeröhrchen mit der zweiten Buchse (170d) verbunden ist, wobei sich die zweite Einstechspitze innerhalb des zweiten Halters (112d) für Probeentnahmeröhrchen erstreckt; und wobei Schritt (b) das Zusammensetzen der ersten Buchse (70d) mit der zweiten Buchse (170d) einschließt, um den ersten und den zweiten Halter (12d, 112d) für Probeentnahmeröhrchen zu verbinden.

14. Das Verfahren nach Anspruch 13, ferner enthaltend:
c) Verpacken des ersten und des zweiten Halters (12d, 112d) für Probeentnahmeröhrchen, die durch die Kupplung verbunden sind.

15. Das Verfahren nach Anspruch 14, ferner enthaltend:
d) Sterilisieren des ersten und des zweiten Halters (12d, 112d) für Probeentnahmeröhrchen, die durch die Kupplung verbunden sind.

## Revendications

1. Dispositif de transfert de fluide comprenant:
un premier support de tube d'échantillonnage (12d) ayant une ouverture interne (22) pouvant loger un premier tube d'échantillonnage à l'intérieur de cette dernière;
un deuxième support de tube d'échantillonnage (112d) ayant une ouverture interne pouvant loger un deuxième tube d'échantillonnage à l'intérieur de cette dernière; et
un dispositif de couplage comprenant un premier raccord (70d), ayant une extrémité de support, une extrémité de connecteur et une première canule s'étendant à partir de ladite extrémité de support et un deuxième raccord (170d) ayant une extrémité de support, une extrémité de connecteur et une deuxième canule s'étendant à partir de ladite extrémité de support, et un connecteur qui est une structure en forme de tube souple (104d, 108d, 106d) et ayant une première extrémité et une deuxième extrémité, ladite première extrémité dudit connecteur étant en mise en prise de fluide avec ladite extrémité de connecteur dudit premier raccord et ladite deuxième extrémité dudit connecteur étant en mise en prise de fluide avec ladite extrémité de connecteur dudit deuxième raccord, pour assembler ledit premier support de tube d'échantillonnage (12d) et ledit deuxième support de tube d'échantillonnage (112d), ledit dispositif de couplage comprenant une trajectoire de fluide interne s'étendant entre une première extrémité et une deuxième extrémité, ladite première extrémité comprenant une première pointe de perforation s'étendant dans ladite ouverture interne dudit premier support de tube d'échantillonnage (12d) et ladite deuxième extrémité comprenant une deuxième pointe de perforation s'étendant dans ladite ouverture interne dudit deuxième support de tube d'échantillonnage (112d),
dans lequel ledit dispositif de transfert de fluide peut établir une trajectoire pour l'écoulement de fluide entre un premier tube d'échantillonnage logé à l'intérieur de ladite ouverture interne dudit premier support de tube d'échantillonnage (12d) et un deuxième tube d'échantillonnage logé à l'intérieur de ladite ouverture interne dudit deuxième support de tube d'échantillonnage (112d) par l'intermédiaire dudit dispositif de couplage.

2. Dispositif de transfert de fluide selon la revendication 1, dans lequel lesdites première et deuxième canules sont fixées à l'intérieur d'une voie de passage interne desdits premier et deuxième raccords (70d, 170d) par des moyens mécaniques ou adhésifs.

3. Dispositif de transfert de fluide selon la revendication 1, dans lequel ladite première extrémité de support dudit premier raccord comprend une structure pour la mise en prise avec ledit premier support de tube d'échantillonnage et ladite extrémité de support dudit deuxième raccord comprend une structure pour la mise en prise avec ledit deuxième support de tube d'échantillonnage.

4. Dispositif de transfert de fluide selon la revendication 3, dans lequel :
ledit premier support de tube d'échantillonnage (12d) comprend des filetages internes et ladite première extrémité dudit premier raccord (70d) comprend des filetages externes pour la mise en prise avec lesdits filetages internes dudit premier support de tube d'échantillonnage, et
ledit deuxième support de tube d'échantillonnage (112d) comprend des filetages internes et ladite deuxième extrémité dudit deuxième raccord (170d) comprend des filetages externes pour la mise en prise avec lesdits filetages internes dudit deuxième support de tube d'échantillonnage (112d).

5. Dispositif de transfert de fluide selon la revendication 3, dans lequel ladite extrémité de support dudit premier raccord (70d) est fixée audit premier support de tube d'échantillonnage (12d) de sorte que la force requise pour que ladite première extrémité de ladite canule perce une butée du premier tube d'échantillonnage logé à l'intérieur de ladite ouverture interne dudit premier support de tube d'échantillonnage (12d) est inférieure à la force requise pour détacher ladite première extrémité dudit premier raccord (70d) dudit premier support de tube d'échantillonnage (12d), et ladite extrémité de support dudit deuxième raccord (170d) est fixée sur ledit deuxième support de tube d'échantillonnage (112d) de sorte que la force requise pour que ladite extrémité de support de ladite canule perce une butée d'un deuxième tube d'échantillonnage logé à l'intérieur de ladite ouverture interne dudit deuxième support de tube d'échantillonnage (112d) est inférieure à la force requise pour détacher ladite extrémité de support dudit raccord (170d) dudit deuxième support de tube d'échantillonnage (112d).

6. Dispositif de transfert de fluide selon la revendication 3, dans lequel ladite extrémité de support dudit premier raccord (70d) est fixée de manière adhésive audit premier support de tube d'échantillonnage (12d) et ladite extrémité de support dudit deuxième raccord (170d) est fixée de manière adhésive audit deuxième support de tube d'échantillonnage (112d).

7. Dispositif de transfert de fluide selon la revendication 1, comprenant en outre :
un premier manchon élastomère s'étendant autour d'une première extrémité de ladite première canule et à l'intérieur de ladite ouverture interne dudit premier support de tube d'échantillonnage (12d) ; et
un deuxième manchon élastomère s'étendant autour d'une deuxième extrémité de ladite deuxième canule et à l'intérieur de ladite ouverture interne dudit deuxième support de tube d'échantillonnage (112d).

8. Dispositif de transfert de fluide selon la revendication 1, dans lequel ledit premier support de tube d'échantillonnage (12d) et ledit deuxième support de tube d'échantillonnage (112d) sont dimensionnés afin de loger les premier et deuxième tubes d'échantillonnage respectifs ayant un diamètre maximum d'environ 16 millimètres.

9. Dispositif de transfert de fluide selon la revendication 1, dans lequel :
ladite première extrémité (70d) dudit raccord comprend des filetages externes configurés pour mettre en prise des filetages internes dudit premier support de tube d'échantillonnage (12d), et
ladite deuxième extrémité (170d) dudit raccord comprend des filetages externes configurés pour mettre en prise les filetages internes du deuxième support de tube d'échantillonnage (112d).

10. Dispositif de transfert de fluide selon la revendication 1, comprenant en outre :
un manchon perforable s'étendant autour de ladite première pointe de perforation,
un manchon perforable s'étendant autour de ladite deuxième pointe de perforation.

11. Procédé pour transférer des fluides corporels entre un premier tube d'échantillonnage et un deuxième tube d'échantillonnage comprenant les étapes suivantes consistant à :
a) prévoir un dispositif de transfert de fluide comprenant des premier et deuxième supports de tube d'échantillonnage (12d, 112d) pour loger des premier et deuxième tubes d'échantillonnage à l'intérieur de ces derniers, lesdits premier et deuxième supports de tube d'échantillonnage (12d, 112d) étant assemblés par le biais d'un dispositif de couplage comprenant un premier raccord (70d), ayant une extrémité de support, une extrémité de connecteur et une première canule s'étendant à partir de ladite extrémité de support et un deuxième raccord (170d) ayant une extrémité de support, une extrémité de connecteur et une deuxième canule s'étendant à partir de ladite extrémité de support, et un connecteur ayant une première extrémité et une deuxième extrémité, qui est une structure en forme de tube souple, ladite première extrémité dudit connecteur étant en mise en prise de fluide avec ladite extrémité de connecteur dudit premier raccord et ladite deuxième extrémité dudit connecteur étant en mise en prise de fluide avec ladite extrémité de connecteur dudit deuxième raccord, la première canule ayant une première pointe de perforation et la deuxième canule ayant une deuxième pointe de perforation de sorte que ladite première pointe de perforation s'étend dans ledit premier support de tube d'échantillonnage (12d) et ladite deuxième pointe de perforation s'étend dans ledit deuxième support de tube d'échantillonnage (112d), ladite deuxième pointe de perforation comprenant un manchon perforable s'étendant autour de cette dernière, et fournissant lesdits premier et deuxième supports de tube d'échantillonnage (12d, 112d) pour loger les premier et deuxième tubes d'échantillonnage à l'intérieur de ces derniers, et
assembler lesdits premier et deuxième supports de tube d'échantillonnage (12d, 112d) par le biais dudit dispositif de couplage de sorte que ladite première pointe de perforation de ladite première canule s'étend dans ledit premier support de tube d'échantillonnage (12d) et ladite deuxième pointe de perforation de ladite deuxième canule s'étend dans ledit deuxième support de tube d'échantillonnage (112d) ;
b) insérer un premier tube d'échantillonnage ayant une fermeture perforable à l'intérieur dudit premier support de tube d'échantillonnage (12d) et perforer ladite fermeture dudit premier tube d'échantillonnage avec ladite première pointe de perforation à l'intérieur dudit premier support de tube d'échantillonnage (12d) ; et
c) insérer un deuxième tube d'échantillonnage ayant une fermeture perforable à l'intérieur dudit deuxième support de tube d'échantillonnage (112d) et perforer ladite ouverture dudit deuxième tube d'échantillonnage avec ladite deuxième pointe de perforation à l'intérieur dudit deuxième support de tube d'échantillonnage (112d) ;
dans lequel l'écoulement de fluide est établi entre ledit premier tube d'échantillonnage et ledit deuxième tube d'échantillonnage par ladite canule, transférant ainsi le fluide entre ledit premier tube d'échantillonnage et ledit deuxième tube d'échantillonnage.

12. Procédé selon la revendication 11, dans lequel les première et deuxième pointes de perforation comprennent un manchon perforable s'étendant autour de ces dernières.

13. Procédé pour assembler un dispositif de transfert de fluide comprenant les étapes suivantes consistant à :
a) prévoir les premier et deuxième supports de tube d'échantillonnage (12d, 112d) pour loger les premier et deuxième tubes d'échantillonnage à l'intérieur de ces derniers ; et
b) assembler lesdits premier et deuxième supports de tube d'échantillonnage (12d, 112d) par le biais d'un dispositif de couplage,
dans lequel ledit dispositif de couplage comprend un premier raccord (70d) ayant une extrémité de support, une extrémité de connecteur et une première canule s'étendant à partir de ladite extrémité de support, ladite première canule ayant une première pointe de perforation avec un premier manchon perforable s'étendant autour de cette dernière, et un deuxième raccord (170d) ayant une extrémité de support, une extrémité de connecteur et une deuxième canule s'étendant à partir de ladite extrémité de support, ladite deuxième canule ayant une deuxième pointe de perforation avec un deuxième manchon perforable s'étendant autour de cette dernière, et un connecteur ayant une première extrémité et une deuxième extrémité, qui est une structure en forme de tube souple, ladite première extrémité dudit connecteur étant en mise en prise de fluide avec ladite extrémité de connecteur dudit premier raccord et ladite deuxième extrémité dudit connecteur étant en mise en prise de fluide avec ladite extrémité de connecteur dudit deuxième raccord, et dans lequel ladite étape a) comprend l'étape consistant à prévoir ledit premier support de tube d'échantillonnage (12d) assemblé avec ledit premier raccord (70d) avec ladite première pointe de perforation qui s'étend à l'intérieur dudit premier support de tube d'échantillonnage (12d) et prévoir ledit deuxième support de tube d'échantillonnage (112d) assemblé avec ledit deuxième raccord (170d) avec ladite deuxième pointe de perforation qui s'étend à l'intérieur dudit deuxième support de tube d'échantillonnage (112d) ; et ladite étape b) comprend l'étape consistant à coupler ledit premier raccord (70d) avec ledit deuxième raccord (170d) pour assembler lesdits premier et deuxième supports de tube d'échantillonnage (12d, 112d).

14. Procédé selon la revendication 13, comprenant en outre l'étape consistant à :
c) emballer lesdits premier et deuxième supports de tube d'échantillonnage (12d, 112d) assemblés par le biais dudit dispositif de couplage.

15. Procédé selon la revendication 14, comprenant en outre l'étape consistant à :
d) stériliser lesdits premier et deuxième supports de tube d'échantillonnage (12d, 112d) assemblés par l'intermédiaire dudit dispositif de couplage.
